# EUROPEAN PATENT APPLICATION

(11) **EP 3 006 037 A1**
(43) Date of publication of application: **13.04.2016**
(21) Application number: 15190917.3
(22) Date of filing: 30.03.2012
(51) Int. Cl.: A61K 38/13, A61P 31/20, A61P 31/14, A61P 1/16

(54) **TREATMENT FOR INFECTION WITH HEPATITIS B VIRUS ALONE OR IN COMBINATION WITH HEPATITIS DELTA VIRUS AND ASSOCIATED LIVER DISEASES**

(30) Priority: 01.04.2011 US 201161470666 P
(62) Divisional of application: 15152496.4
(71) Applicant: Novartis AG, 4056 Basel (CH)
(72) Inventor: NAOUMOV, Nikolai, CH4002 BASEL (CH)
(74) Representative: Rudge, Sewkian

(57) **Abstract**

The invention concerns the use of cyclophilin inhibitors (such as alisporivir or DEB025) in the treatment of Hepatitis B and Hepatitis D virus infections, optionally in combination with interferon or telbivudine, lamivudine, emtricitabine, entecavir, adefovir or tenofovir.

SEQUENCE LISTING

<110>  Novartis AG

<120>  TREATMENT FOR INFECTION WITH HEPATITIS B VIRUS ALONE OR IN
       COMBINATION WITH HEPATITIS DELTA VIRUS AND ASSOCIATED LIVER
       DISEASES

<130>  PAT054611-EP-EPT

<140>  PCT/EP2012055857
<141>  2012-03-30

<150>  US 61/470,666
<151>  2011-04-01

<160>  4

<170>  PatentIn version 3.5

<210>  1
<211>  17
<212>  DNA
<213>  Artificial Sequence

<220>
<223>  Primer

<400>  1
gcatggtccc agcctcc                                                    17


<210>  2
<211>  17
<212>  DNA
<213>  Artificial Sequence

<220>
<223>  Primer

<400>  2
tcttcgggtc ggcatgg                                                    17


<210>  3
<211>  20
<212>  DNA
<213>  Artificial Sequence

<220>
<223>  Probe

<400>  3
atgcccag gtcggac                                                      15


<210>  4
<211>  17
<212>  DNA
<213>  Artificial Sequence

<220>
<223>  primer

<400> 4
gcatggcccc agcctcc 17

## Description

### Background of the Disclosure

The present disclosure relates to non-immunosuppressive cyclosporin analogues which bind to cyclophilins, which are cyclophilin inhibitors, in particular to their pharmaceutical use in the treatment of infection with Hepatitis B virus (HBV) alone or in combination with Hepatitis Delta virus (HDV) and liver diseases caused by such infections.

The cyclosporins and the non-immunosuppressive analogues comprise a class of structurally distinctive, cyclic, poly-N-methylated undecapeptides, commonly possessing pharmacological, in particular immunosuppressive, or anti-inflammatory activity. The first of the cyclosporins to be isolated was the naturally occurring fungal metabolite Ciclosporin or Cyclosporine, also known as cyclosporin A (CsA). Cyclosporins which bind strongly to cyclophilin but are not immunosuppressive have been identified. PCT/EP 2004/009804, WO 2005/021028, or WO 2006/071619 (which are incorporated by reference herein in their entireity) disclose non-immunosuppressive cyclosporins which bind to cyclophilin and have also been found to have an inhibitory effect on Hepatitis C virus (HCV). WO 2006/038088, incorporated herein by reference in its entirety, describes methods and compositions for the use of alisporivir in the treatment of HCV. Alisporivir (DEB025 or Debio-025) is a cyclophilin (Cyp) inhibitor and its mode of action as an anti-HCV agent is via inhibition of host proteins, in particular of cyclophilin A, that are directly involved in HCV replication.

Hepatitis B virus (HBV) is the smallest human DNA virus (1). The HBV genome is partially double-stranded, circular DNA with overlapping reading frames that encode the HBV proteins: envelope proteins - i) small, Hepatitis B surface antigen (HBsAg); ii) middle - preS2 plus HBsAg; iii) large - preS1 plus preS2 plus HBsAg; nucleocapsid protein, hepatitis B core antigen (HBcAg). Hepatitis B e antigen (HBeAg) is a non-structural protein produced during the HBV replication which shares 90% amino acids with the nucleocapsid HBcAg. Eight genotypes of HBV, designated A to H, have been determined, each having a distinct geographical distribution. The virus is non-cytopathic, with virus-specific cellular immunity being the main determinant for the outcome of exposure to HBV - acute infection with resolution of liver diseases with 6 months, or chronic HBV infection that is frequently associated with progressive liver injury. Detection of HBsAg in the serum by conventional diagnostic immunoassays, is the key diagnostic marker for infection with HBV and persistent detection of HBsAg in serum for more than 6 months is the hallmark chronic HBV infection (2,3,4). The best marker for clinically significant HBV replication is the level of HBV DNA in serum, as detected by senistive polymerase chain reaction (PCR)-based assay.

Worldwide more than 350 million people are chronically infected with HBV and are thus at increased risk of developing serious liver disease - such as chronic hepatitis, cirrhosis, liver failure and hepatocellular carcinoma (HCC). The natural evolution of chronic HBV infection includes four consecutive phases: (1) *early 'immunotolerant' phase* - high levels of virus replication and minimal liver inflammation; (2) *immune reactive phase* - significant hepatic inflammation and elevated serum aminotransferases; with some patients progressing to (3) *'non-replicative' phase* - seroconversion to anti-HBe; undetectable or low level of viremia (below 2000 IU/ml by PCR-based assays); resolution of hepatic inflammation; and (4) *HBeAg-negative chronic hepatitis B -* due to the emergence of specific viral mutations, which prevent the production of HBeAg but do not hamper virus replication. This form of CHB is characterized by fluctuating serum HBV DNA and serum aminostransferases (ALT and AST) levels, and progressive liver disease. It is important to note that chronic hepatitis B (CHB) may present either as hepatits B e antigen (HBeAg)-positive or HBeAg-negative CHB. Longitudinal studies of patients with CHB indicate that the 5-year cumulative incidence of developing cirrhosis ranges from 8 to 20%. The 5-year cumulative incidence of hepatic decompensation is approximately 20% (2). The worldwide incidence of HCC has increased and presently constitutes the fifth most common cancer (2,3,4). The annual incidence of HBV-related HCC is high ranging from 2-5% when cirrhosis is established (2).

The primary goal of treatment for CHB is to permanently suppress HBV replication and improve liver disease. Clinically important short-term goals are to achieve HBeAg-seroconversion, normalization of serum ALT and AST, resolution of liver inflammation and to prevent hepatic decompensation (2). The ultimate goal of treatment is to achieve durable response to prevent development of cirrhosis, liver cancer and prolong survival. HBV infection can not be eradicated completely due to persistence of a particular form of viral covalently closed circular DNA (ccc HBV DNA) in the nuclei of infected hepatocytes. However, treatment-induced clearance of serum HBsAg is a marker of termination of chronic HBV infection and has been associated with the best long-term outcome (2,3).

Seven drugs are currently available for treatment of CHB - conventional interferon, pegylated interferon and direct antiviral agents. The direct antivirals (nucleos/tide analogues) belong to three classes: L-nucleosides (lamivudine, telbivudine and emtricitabine); deoxyguanosine analogue (entecavir) and nucleoside phosphonates (adefovir and tenofovir) which directly interfere with HBV DNA replication, primarily as chain terminators (2,3,4). In HBeAg-positive patients, virological treatment response rates (undetectable serum HBV DNA) at one year are when treated with: peg-interferon 25%; lamivudine 36-40%; entecavir 67%; telbivudine 60%; tenofovir 74% (2). Loss of HBsAg after one year is very low - between 0 and 3%. In HBeAg-negative patients with CHB the rates of undetectable HBV DNA at one year are when treated with: peg-interferon 63%; lamivudine 72%; entecavir 90%; telbivudine 88%; tenofovir 91%. Loss of HBsAg was 0% with any of the direct antiviral agents. The currently available treatments are suboptimal and there is a need for better therapies to meet the treatment goals in CHB. The key limitations for interferon treatment are major side-effects, low rate of HBV DNA suppression and low rate of ALT normalization; key limitations of the treatment with direct antivirals are: development of resistance; rebound of HBV replication after stopping therapy requiring prolonged, life-long therapy, very low rate of HBsAg clearance, increasing the risk of adverse events with prolonged, life-long therapy.

A proportion of patients with chronic HBV infection (serum HBsAg-positive) will have co-infection with Hepatitis Delta virus (HDV). HDV is a defective virus with a single-stranded circular RNA genome, causing acute or chronic liver diseases only in association with hepatitis B virus (1). The only protein expressed by the HDV RNA is hepatitis Delta antigen, which is the nucleocapsid of the virus. HDV does not encode its own envelope protein (HBsAg) and must "borrow" this from hepatitis B virus. Thus, the infection with HDV occurs only in the presence of HBV - either simultaneously with HBV (co-infection) or subsequently, with the duration of infection determined by that of HBsAg positivity. In a HBsAg-positive patient, active infection with HDV is confirmed by the detection of HDV RNA in serum, immunohistochemical staining for Hepatitis Delta antigen in hepatocytes, or detection of IgM anti-HDV in serum (2).

HDV has worldwide distribution with the highest endemicity in Mediterranean countries, parts of south and middle Africa and in South America. Is it estimated that 5% of all hepatitis B virus carriers are infected with HDV (5). In countries where HBV prevalence is low, such as North America and northern Europe, HDV infection is largely restricted to populations at risk for parenteral HBV exposure like intravenous drug users. Immigration from regions of high HDV endemicity has increased the prevalence in several European countries recently leading approximately 10% amongst chronic HBV patients.

The only approved treatment of chronic hepatitis D is interferon-alpha with unsatisfactory results. Addition of ribavirin to interferon did not improve the response. Direct antivirals that block HBV replication were shown to have no effect on HDV replication. Combinations of interferon plus lamivuidne or interferon plus adefovir did not improve the response compared to interferon alone (2,3,6). Thus, treatment of chronic hepatitis D remains a major unmet medical need as HDV-induced liver damage leads to cirrhosis, liver decompensation and in some cases death due to liver failure.

Therefore it is an object of the present disclosure to provide new methods for the treatment of patients with hepatitis B virus infection alone or patients infected with hepatitis B virus and hepatitis Delta virus and the liver diseases or disorders induced by these infections, such as chronic hepatitis, cirrhosis, hepatic decompensation and HCC.

Surprisingly we have found that cyclophilin inhibitors, in particular alisporivir, have antiviral properties against Hepatitis B virus that can be used effectively in the treatment of HBV infections. In particular, we have found that alisporivir abrogates (or interferes) HBV replication and reduces HBsAg production in liver cells. -. Furthermore, the cyclophilin inhibitors, in particular alisporivir, can also be used in the treatment of chronic hepatitis D.

Accordingly, the present invention provides new anti-HBV and anti-HDV treatments using alisporivir.

Furthermore, the present disclosure provides methods for the treatment of HBV and/or HDV infections and of the induced liver diseases in patients comprising administering an effective amount of a cyclophilin inhibitor, in particular alisporivir, either alone or in combination with a direct antiviral agent or with interferon-alfa. The disclosure further provides alisporivir for use in the treatment of HBV infection and associated liver diseases and also for use in the treatment of patients infected with HBV and HDV and associated liver disease.

### Summary of the Disclosure

Further the following is described:
1.1 A method for treating Hepatitis B virus infections and HBV-induced disorders in a patient, comprising administering to said patient alisporivir.
1.2 A method for inhibiting HBV replication and HBsAg production, comprising administering alisporivir.
1.3. A method for treating Hepatitis Delta virus infections and HDV-induced disorders in a patient, comprising administering to said patient alisporivir.
1.4. A method for inhibiting HDV replication and HBsAg production, comprising administering alisporivir.
1.5. A method for reducing HBV or HDV-induced liver damage, and for reduced liver cell death comprising administering alisporivir.
1.6. A method for normalization of serum ALT levels comprising administering alisporivir.
1.7. A method for reduction or resolution of liver inflammation comprising administering alisporivir.
2. Use of alisporivir in the preparation of a pharmaceutical composition for use in any method as defined above.
3. Use of alisporivir in the preparation of a medicament for use in any method as defined above.
4. Use of alisporivir in combination with a direct antiviral agent that inhibits HBV replication in the preparation of a medicament for use in any method as defined above.
5. Use of alisporivir in combination with an interferon for treatment of patients with HBV infections and associated liver disease, and for patients infected with HBV and HDV and associated liver disease.

### Brief description of the Figures

Figure 1 shows that the significant reduction of intracellular HBV DNA levels (by 10-fold at 72 hours) was observed with alisporivir at a concentration of 5 micrograms/mL.
   As it can bee seen in Figure 2, alisporivir at a concentration of 5.0 micrograms/mL showed a greater effect in reducing the HBV DNA levels in cell culture supernatants when compared with the effect of NIM811.

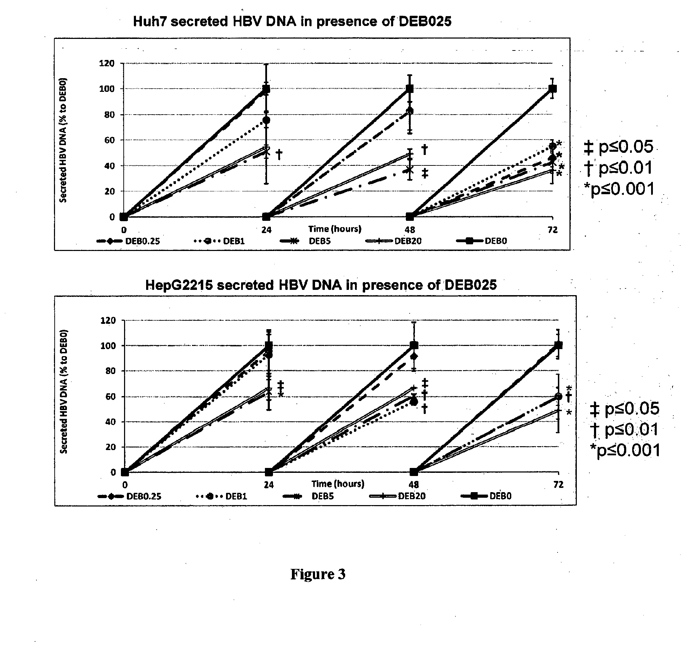

Figure 3 illustrates the dose-dependent reduction of HBV DNA secreted from transiently transfected cells (Huh7) or from stably transfected (HepG2215) cells in presence of DEB025.

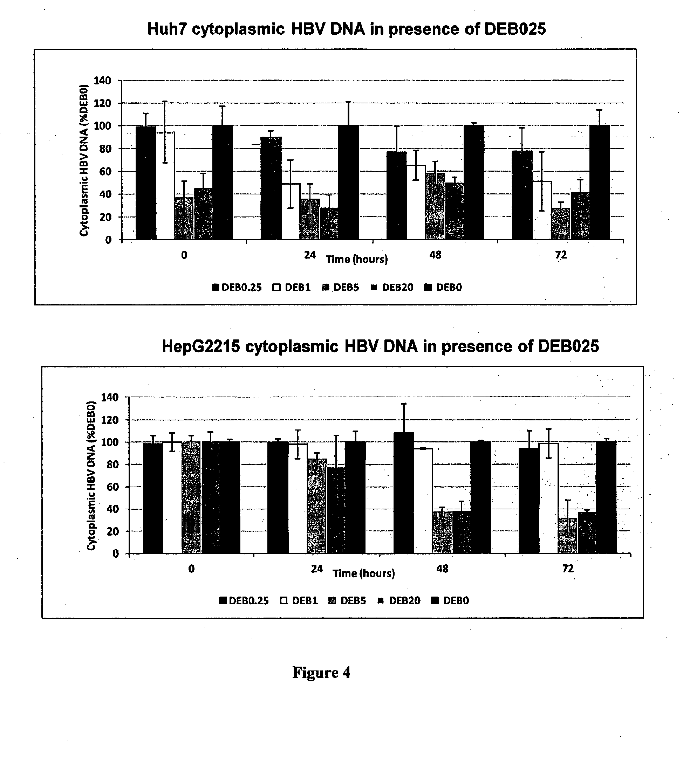

Figure 4 illustrates dose dependent reductions of cytoplasmic HBV DNA in Huh7 and HepG2215 cells in presence of DEB025.

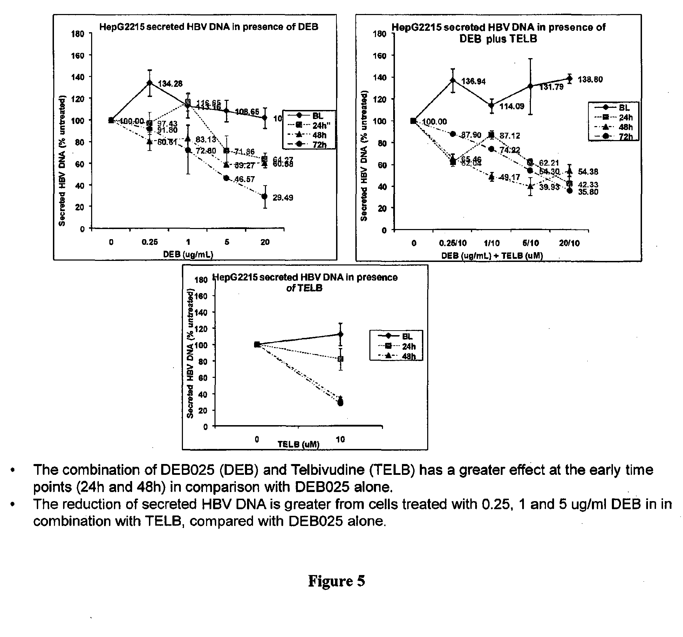

Figure 5 illustrates reductions of secreted HBV DNA in HepG2215 cells in presence of DEB025 (DEB), telbivudine (TELB) and DEB + TELB.

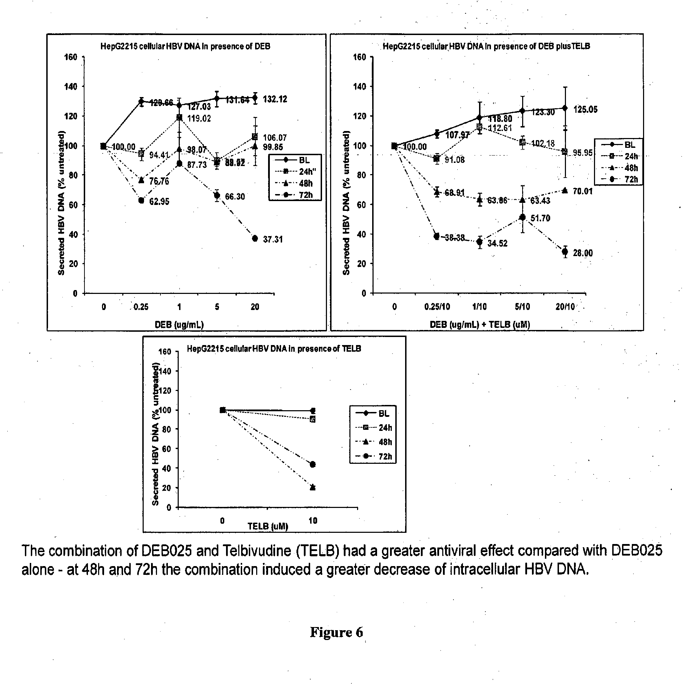

Figure 6 illustrates reductions of cellular HBV DNA in HepG2215 cells in presence of DEB025 (DEB), telbivudine (TELB) and DEB + TELB.

### Description of the Disclosure

As used herein a "Hepatitis B virus infected patient" means a patient being infected with any Hepatitis B virus genotype, e.g., genotype A, B, C, D etc. In some embodiments, the patient is infected with Hepatitis delta virus. In some embodiments, the Hepatitis B virus infected patient may be infected also with Hepatitis delta virus.

In the present invention, an interferon may be pegylated or non-pegylated and may include interferons such as: Intron-A®, interferon alfa-2b (Schering Corporation, Kenilworth, NJ); PEG-lntron®, peginteferon alfa-2b (Schering Corporation, Kenilworth, NJ); Roferon®, recombinant interferon alfa-2a (Hoffmann-La Roche, Nutley, NJ); Pegasys®, peginterferon alfa-2a (Hoffmann-La Roche, Nutley, NJ); Berefor®, interferon alfa 2 available (Boehringer lngelheim Pharmaceutical, Inc., Ridgefield, CT); Sumiferon®, a purified blend of natural alpha interferons (Sumitomo, Japan); Wellferon®, lymphoblastoid interferon alpha nl (GlaxoSmithKline); Infergen®, consensus alpha interferon (InterMune Pharmaceuticals, Inc., Brisbane, CA and Amgen, Inc., Newbury Park, CA); Alferon®, a mixture of natural alpha interferons (Interferon Sciences, and Purdue Frederick Co., CT); Viraferon®; and combinations of these interferons.

Conjugated interferons that may be used include, for example, Albuferon (Human Genome Science) which is conjugated to human albumin. Interferon conjugated to a water-soluble polymer or polyalkylene oxide homopolymers such as polyethylene glycol (PEG) or polypropylene glycols, polyoxyethylenated polyols, copolymers thereof and block copolymers thereof. As an alternative to polyalkylene oxide-based polymers, effectively non-antigenic materials such as dextran, polyvinyl pyrrolidones, polyacrylamides, polyvinyl alcohols, carbohydrate-based polymers and the like can be used. Interferon-polymer conjugates are described in US 4766106, US 4917888, EPA 0 236 987, EPA 0 510 356 and WO 95/13090. Since the polymeric modification sufficiently reduces antigenic responses, the foreign interferon need not be completely autologous. Interferon used to prepare polymer conjugates may be prepared from a mammalian extract, such as human, ruminant or bovine interferon, or recombinantly produced. Other forms of interferons include interferon beta, gamma, tau and omega, such as Rebif (Interferon beta 1a) by Serono, Omniferon (natural interferon) by Viragen, or Omega Interferon by Boehringer Ingelheim. Oral interferons such as oral interferon alpha by Amarillo Biosciences.

Additional examples of interferons that may be used include pegylated interferon alpha, for example pegylated interferon α-2a, pegylated interferon α-2b, pegylated consensus interferon or pegylated purified interferon-α product. Pegylated interferon α-2a is described in European Patent 593,868 (incorporated herein by reference in its entirety) and commercially available e. g. under the trade name PEGASYS® (Hoffmann-La Roche). Pegylated interferon-α-2b is described, e.g. in European Patent 975,369 (incorporated herein by reference in its entirety) and commercially available e.g. under the trade name PEG- INTRON A® (Schering Plough). Pegylated consensus interferon is described in WO 96/11953 (incorporated herein by reference in its entirety).

In preferred embodiments, the interferon used in the methods of the invention is pegylated interferon. In other embodiments, the interferon is selected from the group consisting of interferon alpha-2a, Interferon alpha-2b, a consensus interferon, a purified interferon alpha product or a pegylated interferon alpha-2a, pegylated interferon alpha-2b, and pegylated consensus interferon, a mixture of natural alpha and combinations thereof.

Preferably the methods using interferon alpha use a pegylated interferon alpha-2b and the amount of pegylated interferon alpha-2b is from 0.5 to 2.0 micrograms/kilogram per week on a weekly, three times a week, every other day or daily basis.

As used herein, "microgram/kilogram" means microgram drug per kilogram body weight of the mammal - including man - to be treated.

As used herein, the term "treatment" or "treat" refer to both prophylactic or preventative treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment.

By "therapeutic regimen" is meant the pattern of treatment of an illness, e.g., the pattern of dosing used during HBV therapy. A therapeutic regimen may include an induction regimen and a maintenance regimen.

The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both.

The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, the term "about", unless the context dictates otherwise, is used to mean a range of + or - 10%.

In other embodiments, the interferon alpha is a pegylated interferon alpha-2a and the amount of pegylated interferon alpha-2a administered is from 20 to 250 micrograms/kilogram per week on a weekly, three times a week, every other day or daily basis. Preferably, the interferon peg-IFNa2a is administered at an amount of 180 micrograms once per week.

In specific embodiments, the exemplary interferon used in the methods herein is interferon selected from the group consisting of Intron-A®; PEG-lntron®; Roferon®; Pegasys®; Berefor®; Sumiferon®; Wellferon®; Infergen®; Alferon®; Viraferon®; Albuferon® (Human Genome Science); Rebif; Omniferon; Omega and combinations thereof.

In some embodiments, alisporivir is used in the treatment of Hepatitis B virus infection in a patient and/or in the treatment of Hepatitis D virus infection in a patient. In still another aspect, alisporivir is administered in combination with a direct antiviral agent or an interferon.

In some other embodiments it is described a method for treating a Hepatitis B virus infection in a patient comprising administering an effective amount of alisporivir and optionally administering to the patient an interferon or a direct antiviral agent. In still an other aspect, alisporivir is for use in improvement of liver inflammation, reduce liver cell death (as assessed by ALT levels) and prevention of progression of liver disease.

In another embodiment, a pharmaceutical composition comprising alisporivir for use according to any of the methods disclosed herein and a package comprising said pharmaceutical composition in combination with instructions to administer said composition is described.

In another embodiment, alisporivir may be administered with additional agents of the standard of care that promote the antiviral efficacy of the therapy treatment.

Direct antiviral agent, is used herein to mean agents that interfere with specific steps in the hepatitis B virus (HBV) replication cycle. A direct antiviral agent that inhibits HBV replication may be for example any of the currently anti-HBV agents approved for the treatment of HBV, i.e. telbivudine, lamivudine, emtricitabine, entecavir, adefovir and tenofovir.

In treatment described above effective dosages of the standard of care agents are administered in compositions, i.e. they may be administered together (i.e., simultaneously), but may also be administered separately or sequentially. In general, combination therapy is typically administered together, the rationale being that such simultaneous administration induces multiple simultaneous stresses on the virus. The specif dosages given will depend on absorption, inactivation and excretion rate of the drugs as well as other factors. It is to be noted that dosage values will also vary with the severity of the condition to be alleviated. The terms "co-administration" or "combined administration" or "administered in combination with" or the like as utilized herein are meant to encompass administration of the selected therapeutic agents to a single patient, and are intended to include treatment regimens in which the agents are not necessarily administered by the same route of administration or at the same time. Fixed combinations are also within the scope of the present disclosure. The administration of a pharmaceutical combination of the disclosure results in a beneficial effect, e.g. a synergistic or additive therapeutic effect, compared to a monotherapy applying only one of its pharmaceutically active ingredients or as compared to the current standard of care therapy. The treatment used in the methods described herein may be administered by any conventional route. One or more components may be administered parentally, e.g., in the form of injectable solutions or suspensions, or in the form of injectable deposit formulations. Preferably, alisporivir will be administered orally in the form of capsules, tablets or solutions or suspensions for drinking. Pharmaceutical compositions for oral administration comprising alisporivir typically further comprise one or more pharmaceutically acceptable carrier substances. Typically, these compositions are concentrated and need to be combined with an appropriate diluent, e.g., water, prior to administration. Pharmaceutical compositions for parenteral administration typically also include one or more excipients. Optional excipients include an isotonic agent, a buffer or other pH- controlling agent, and a preservative. These excipients may be added for maintenance of the composition and for the attainment of preferred ranges of pH (about 6.5-7.5) and osmolarity (about 300 mosm/L).

The administration of alisporivir as described herein is in a single dose form or in more than one dosage form; one or more oral dosage forms may be administered at each time per day. In some embodiments, alisporivir is administered in doses of 200 mg to 1000 mg.

The efficacy of the therapy regimen may be monitored using standard protocols. Treatment may be followed by determinations of HBV levels in serum and measurement of serum ALT levels. For example, the patients may be assessed for the presence of HBV DNA in their serum. HBV DNA (IU/mL) can be measured at regular intervals during the treatment, e.g., at Day 1 (pre-dose and 4, 8, and 12 hours post-dose) and pre-dose at Day 2, Day 3, Day 8, Day 15, Day 29, and at Week 12, Week 24, Week 36, Week 48, Week 72 (when applicable), and at follow up.

The efficacy of therapy will be monitored using internationally accepted parameters (2,3,4):
1.1. Monitoring response in patients with Hepatitis B virus
   a) Serum HBV DNA levels are monitored using sensitive quantitative PCR-based assays to assess the effect on viral replication.
   b) In HBeAg-positive patients - HBeAg is monitored along with the corresponding anti-HBe to determine whether HBe-seroconversion has occurred.
   c) Serum levels of ALT and/or AST are monitored to assess impact on liver inflammation and liver cell death
   d) Serum HBsAg is monitored - qualitatively and quantitatively as HBsAg clearance would indicate optimal treatment outcome.
   e) The development of mutations in the HBV genome that confer resistance to the treatment used
1.2 Monitoring response in patients with Hepatitis Delta virus. In addition to the above listed markers for HBV infection, the following HDV-specific test will be used:
   a) Serum HDV RNA levels are monitored to assess the effect on hepatitis Delta virus replication.
   b) Detection of Delta antigen by immunohistochemitry in liver tissue may provide additional information indicating ongoing HDV-replication.

The following Examples illustrate the invention described hereinbefore.

### EXAMPLES

### Experimental design and human cell lines:

The experiments have used several human hepatoma-derived cell lines that have been established as models for in vitro studies on hepatitis B virus or hepatitis delta virus life cycle and to evaluate the impact of various compounds on viral replication and viral protein production - HepG2 and HuH-7 (HBV negative), PLC/PRF/5 (HBV positive, producing HBsAg only), and HepG2.2.15 (HBV positive, supporting full HBV replication).

### A) Investigation of antiviral activities of cyclophilin inhibitors, in particular DEB025 (Alisporivir), on hepatitis B virus replication and HBsAg production.

HepG2215 cell line, derived from HepG2 cells, was stably transfected with HBV DNA and supported full HBV replication with production of infectious virions and HBsAg. HepG2215 cells were cultured in 12-well plates, as described previously (7, 8), for 7 days prior to adding the cyclophilin inhibitors.

The cells were treated with 0.25, 1.0 or 5.0 micrograms/mL of NIM811 or DEB025; cells and supernatants were harvested separately at baseline, 6, 24, 48 and 72 hours after the addition of NIM811 or DEB025, respectively.

To assess the role of cyclophilin A in HBV replication, HepG2215 cells were transfected with siRNA for cyclophilin A and incubated with medium only or with the addition of NIM811 or DEB025.Similarly, the role of Cyclophilin B, C and D in HBV and HDV replication will be evaluated by siRNA specific for CypB, C and D.

HuH-7 cells were transfected with a plasmid containing a pBlueScript KS(+) vector with a 1.5 HBV DNA genomes of subtype adw2 was used. Subconfluent HuH-7 cells were transfected with 5 µg of plasmid DNA per 60-mm-diameter dish using 10 µl of Superfect reagent (Qiagen, Crawley, UK). The supernatants were replaced 18 h posttransfection with medium supplemented with 10% FCS, and the cells were cultured for 3 days, as described (7).

PLC/PRF/5 cells (Alexander cell line) contains integrated fragment of HBV DNA and produces HBsAg only, but does not support full HBV replication (9). This is analogous to the hepatocytes in patients that are inactive HBsAg carriers (i.e the non-replicative phase of chronic HBV infection). This cell line was employed to assess specifically the impact of cyclophilin inhibitors on HBsAg production separate from full virion production.

### Results

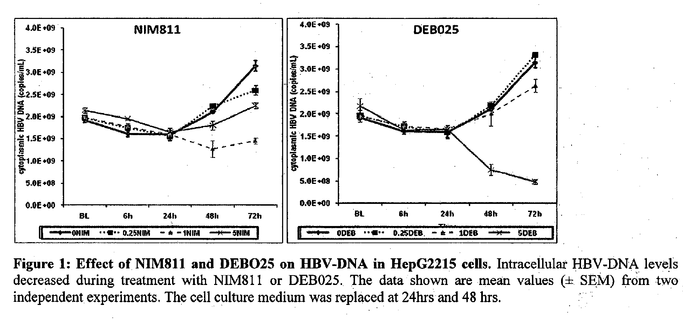

1) Figure 1: Both cyclophilin inhibitors NIM811 and DEB025 reduced intracellular, HBV nucleocapsid-associated HBV DNA levels. The HBV DNA reduction was between 2 and 10-fold as compared to the HepG2215 cells in the absence of NIM811 or DEB025. The most pronounced reduction of intracellular HBV DNA levels (by 10-fold at 72 hours) was observed with DEB025 (5 micrograms/mL), which was greater than the reduction observed with NIM811 at 72h.

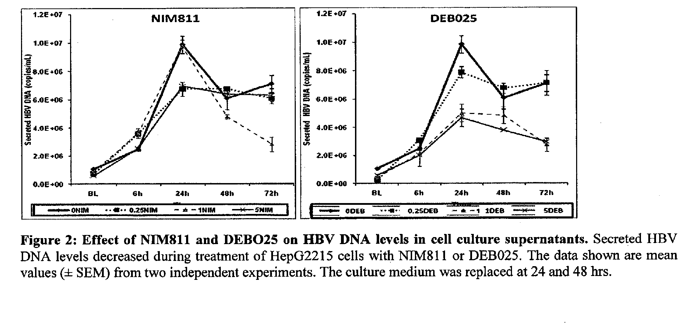

2) Figure 2: DEB025 (at 5.0 micrograms/mL) showed a greater effect in reducing the HBV DNA levels in cell culture supernatants when compared with the effect of NIM811.

### B) Investigation of antiviral activities of cyclophilin inhibitors, in particular DEB025 (Alisporivir), on hepatitis Delta virus replication.

Infection with the Hepatitis delta virus (HDV) is always associated with the presence of the helper virus - hepatitis B virus. The basis for this dependence is that the HBV envelope proteins are required for both HDV entry into hepatocytes and the assembly of new HDV particles. Once inside a susceptible cell, the single-stranded circular RNA genome of HDV can replicate by RNA-directed RNA synthesis using redirection of a host RNA polymerase. In vitro, it is possible to study HDV genome replication in cultured cell lines in the absence of the helper HBV. For example, replication is initiated when cells are transfected with a cDNA version of the HDV sequence (10). In addition, the need can be met by cotransfecting the HDV RNA along with an mRNA for δAg (11).

### C) Investigation of antiviral activities of cyclophilin inhibitors, in particular DEB025 (Alisporivir) in combination a direct anti-viral targeting HBV-DNA polymerase (telbivudine), on hepatitis B virus replication.

Stably transfected (HepG2215) and transiently transfected (HUH-7) cells, producing full HBV virions and HBsAg particles, were treated with a range of Alisporivir concentrations (0.25, 1.0 5.0 or 20 micrograms/ml) alone, telbivudine alone, or combinations of Alisporivir and telbivudine. To determine the involvement of individual cyclophilins, HepG2215 cells were transfected with siRNA-specific for cyclophilin (Cyp) A, C or D and additionally treated with Alisporivir. Cytoplasmic extracts and supernatants were harvested at baseline; 24, 48 and 72 hours post-treatment. The kinetics of antiviral activity was assessed by quantitation of intracellular and secreted HBV-DNA (real-time qPCR) and HBsAg levels (ELISA).

Both in HepG2215 and HUH-7 cells, Alisporivir treatment resulted in dose-dependent reduction of intracellular and secreted HBV-DNA at all time points, by 70% (p=0.004) and 63% (p<0.001), respectively, compared with untreated controls. The combination of Alisporivir and telbivudine had greater effects in reducing intracellular (p=0.001) and secreted (p=0.028) HBV-DNA, and >3-fold reduction of HBsAg versus either Alisporivir or telbivudine alone. CypA, C or D expression was markedly reduced after transfection with corresponding siRNA, which was associated with significant decrease of HBV-DNA and HBsAg levels (p<0.001). Alisporivir treatment of cells silenced for CypA, C or D further reduced HBV-DNA and HBsAg levels, with greater antiviral effects in CypC or CypD silenced cells, compared with CypA silenced cells (p<0.001).

These results suggest that Alisporivir interferes with multiple sites of HBV replication and its antiviral activity is synergistic with direct antiviral targeting viral DNA polymerase, such as telbivudine.

### 1. Cell culture and transfection.

The human hepatoma cell line HuH7 was cultured in Dulbecco's modified Eagle's medium supplemented with 10% fetal bovine serum, penicillin, and streptomycin and was incubated at 37°C in 5% CO₂, as described previosuly (12). For transfection studies, cell cultures were seeded overnight in six-well plates or 60-mm-diameter petri dishes and transfected with 5 and 10 µg, respectively, of 50:50 mixtures of HDAg mRNA and HDV RNA (1.2 times the genome-length) using DMRIE-C reagent (Gibco BRL) according to the manufacturer's directions. Following transfection, the cultures were incubated overnight, the medium was changed, and incubation was continued for up to an additional 2 to 7 days.

### 2. HBV DNA quantitation in cytoplasm and cell supernatants

HBV replication was assessed by quantitation of both viral nucleocapsid-associated HBV DNA (cytoplasmic) and the HBV DNA levels in cell culture supernatants, as described previously (8,9). Nucleocapsid HBV DNA was extracted and quantitated by TaqMan real-time PCR (ABI Prism 7700). Intracellular HBV DNA was normalized with b-actin as a house-keeping gene. Secreted HBV DNA was extracted with QIAmp DNA minikit (Qiagen, Sussex, UK) and also quantitated by TaqMan. The sensitivity of HBV DNA quantitation was verified in each run with serial dilutions of HBV DNA plasmid, as well as the EuroHep HBV DNA standard, as described (9,10).

### 3. HBsAg quantitation

HBsAg levels in cell culture supernatants (from 2215 and PLC/PRF/5 cells) were quantitated with Elisa (Abazyme, Needham, MA) using serial dilutions of known amounts of HBsAg (9).

### 4. SiRNA transfection

HepG2215 cells were seeded with the siRNAs (Dharmacon Inc., Lafayette, CO) at 50% density and incubated for 48h. Subsequently, the cells were washed and NIM811 or DEB025 was added (BL). Cellular and supernatant samples were collected at 6h, 24h (medium was replaced) and at 48hours. Total DNA was extracted from the samples and HBV DNA was quantitated by real time PCR, as above. Total RNA was extracted with phenol, chloroform. The RNA was reverse transcribed with Quantitect Reverse transcription kit (Qiagen) and cDNA quantitated with SYBR Green Quantitect kit using Quantitect primers (Qiagen, Sussex, UK).

### 5. Immunoblot Analysis for HBsAg

After 48 hours, the cells (PLC/PRF/5 or HepG2215 cells) were trypsinized and washed two times in phosphate-buffered saline and resuspended in lysis buffer. The presence of human IgG and HBsAg in PLC/PRF/5 cells was analyzed by western blot in cells cultured in the presence or absence of monoclonal anti-HBs IgG, as described. (7)

### 6. In vitro transcription for hepatits Delta RNA

HDV RNAs 1.2 times the genome length are transcribed from plasmids pBSδ1.2G, pBSδ1.2AG, pBSδ1.2G(2xS), and pBSδ1.2AG(2xS) with T7 MEGAscript kits (Ambion) after linearization with restriction enzyme *Not*I, as decribed previously (13). Capped mRNA for HDAg is transcribed from plasmid pX9-I/II after linearization with *Hin*dIII by using a T7 m-Message m-Machine kit (Ambion). Unlabeled monomer genomic and antigenomic HDV RNAs are transcribed from pTMδSalA and pTMδSalB with T7 MEGAscript after linearization by *Pst*I digestion.

### 7. Northern analysis of HDV RNA

Viral RNA from inocula or sera are purified using a QIAamp Viral RNA mini kit (QIAGEN, Valencia, Calif.) according to the manufacturer's instructions (14,15). Various amounts of RNA are then incubated at 55°C for 50 min in the presence of 1.9 M glyoxal (Fisher Scientific, Fair Lawn, N.J.)-7.1 mM sodium phosphate (pH 6.8)-4.5 mM EDTA-35% DMSO. Samples are then loaded on a 1.5% agarose gel containing 10 mM sodium phosphate, pH 6.8, and subjected to electrophoresis for 4 h at 150 mA. For supernatants, half of the RNA is used, while for cellular RNA, 5 µg is loaded. RNAs are capillary transferred overnight to Zeta-Probe (Bio-Rad, Richmond, Calif.) membranes. After transfer, the membrane is either baked or UV cross-linked using a Stratalinker (Stratagene). The blot is hybridized, as decibed in detail (15). After hybridization, the blot is washed at 70°C with 400 ml of 2x SSPE-0.1% SDS, then with 400 ml of 1 × SSPE-0.1% SDS, and then with 200 ml of 0.1 × SSPE-0.1% SDS. The membrane is dried at 70°C and subjected to autoradiography and phosphorimager (Molecular Dynamics) analysis.

### 8. Real-time PCR quantification in serum samples (as decribed previosuly (16))

HDV RNAs are extracted from 250 µl of serum or plasma by use of a QIAamp MinElute virus vacuum (QIAGEN, Courtaboeuf, France). cDNAs are synthesized as previously described and are purified with Montage PCR centrifugal filter devices (Millipore, Molsheiin, France).

The forward primer is selected to target the ribozyme region of the genome, and the reverse primer targeted region I of the antigenome ribozyme. The probe, which hybridizes to the same region as the reverse primer, is designed to anneal to the antigenomic sequence to avoid base pairing with the reverse primer. The names and sequences of the primers and probe are as follows: Delta-F (forward primer), 5'-GCATGGTCCCAGCCTCC-3'; Delta-R (reverse primer), 5'-TCTTCGGGTCGGCATGG-3'; and Delta-P (probe), 5'-FAM-ATGCCCAGGTCGGAC-MGB-3'. Because of the existence of one mismatch with the sequences of HDV-3 genomes, the following second direct primer is specifically designed for the amplification of HDV-3 isolates: T3-Delta-F, 5'-GCATGGCCCCAGCCTCC-3'. Real-time PCRs are performed by using the TaqMan Universal PCR master mix (Applied Biosystems, Courtaboeuf, France). The reaction consists of one initiating step of 2 min at 50°C, followed by 10 min at 95°C and then 45 cycles of amplification including 15 s at 95°C and 1 min at 60°C. The reactions, data acquisition, and analyses are performed with the ABI PRISM 7000 sequence detection system (Applied Biosystems, Courtaboeuf, France).

### References:

1. Naoumov NV. Hepatitis Viruses (excluding hepatitis C virus). Oxford Textbook of Medicine 5th Edition, Eds. D. Warrell, T. Cox, J. Firth, Oxford University Press, 2010, Vol 1: 609-615.
2. EASL Clinical Practice Guidelines : Management of chronic hepatitis B. J Hepatol 2009; 50:227-242.
3. Lok A, Mcmahon B. Chronic Hepatitis B:Update 2009. AASLD Practice Guidelines. Hepatology 2009;50 (3):1-36.
4. Liaw Y-F et al. Asian-Pacific consensus statement on the management of chronic hepatitis B: a 2008 update. Hepatol Int 2008.
5. Wedemeyer H, Manns MP. Epidemiology, pathogenesis and management of hepatitis D: update and challenges ahead. Nat Rev Gastroenterol Hepatol. 2010;7(1):31-40.
6. Wedemeyer H, et al. Peginterferon plus adefovir versus either drug alone for hepatitis delta. N Engl J Med. 2011;364(4):322-31.
7. R. Schilling, et al. Endocytosis of hepatitis B immune globulin into hepatocytes inhibits the secretion of hepatitis B virus surface antigen and virions. J Viol. 2003;77:8882-92.
8. S. Phillips, et al. CD8(+) T cell control of hepatitis B virus replication: direct comparison between cytolytic and noncytolytic functions. J Immunol. 2010;184:287-95.
9. A. Neumann, et al. Novel mechanism of antibodies to hepatitis B virus in blocking viral particle release from cells. Hepatology 2010;52:875-85.
10. Chao, M., S.-Y. Hsieh, and J. Taylor. 1990. Role of two forms of the hepatitis delta virus antigen: evidence for a mechanism of self-limiting genome replication. J. Virol. 64:5066-5069.
11. Modahl, L. E., and M. M. C. Lai. 1998. Transcription of hepatitis delta antigen mRNA continues throughout hepatitis delta virus (HDV) replication: a new model of HDV RNA transcription and regulation. J. Virol. 72:5449-5456.
12. Macnaughton TB, Lai M. Hepatitis Delta Virus RNA Transfection for the Cell Culture Model Methods in Molecular Medicine, 2004, Volume 96, II, 351-357, DOI: 10.1385/1-59259-670-3:351
13. Macnaughton, TB et al. Rolling Circle Replication of Hepatitis Delta Virus RNA Is Carried Out by Two Different Cellular RNA Polymerases J Virol. 2002; 76(8): 3920-3927.
14. Dingle, K., V. Bichko, H. Zuccola, J. Hogle, and J. Taylor. 1998. Initiation of hepatitis delta virus genome replication. J. Virol. 72:4783-4788.
15. Bruno B. et al. A Prenylation Inhibitor Prevents Production of Infectious Hepatitis Delta Virus Particles J Virol. 2002; 76(20): 10465-10472.
16. Le Gal, F et al. Quantification of Hepatitis Delta Virus RNA in Serum by Consensus Real-Time PCR Indicates Different Patterns of Virological Response to Interferon Therapy in Chronically Infected Patients J Clin Microbiol. 2005; 43(5): 2363-2369.

## Claims

1. Alisporivir for use in the treatment of Hepatitis B virus infection in a patient.

2. Alisporivir for use in the treatment of Hepatitis D virus infection in a patient.

3. Alisporivir for use according to claim 1 or 2, wherein alisporivir is administered in combination with a direct antiviral agent.

4. Alisporivir for use according to claim 1 or 2, wherein the direct antiviral agent is selected from the group consisting essentially of telbivudine, lamivudine, emtricitabine, entecavir, adefovir and tenofovir.

5. Alisporivir for use according to claim 1 or 2, wherein alisporivir is administered in combination with an interferon.

6. Alisporivir for use in the treatment of Hepatitis B virus infection and Hepatitis D virus infection in a patient.

7. Alisporivir for use according to claim 6, wherein alisporivir is administered in combination with a direct antiviral agent or an interferon.

8. A method for treating a Hepatitis B virus infection in a patient comprising administering an effective amount of alisporivir and optionally administering to the patient an interferon or a direct antiviral agent.

9. A method for treating a Hepatitis Delta virus infected infection in a patient comprising administering an effective amount of alisporivir and optionally administering to the patient an interferon or a direct antiviral agent.

10. A method for prevention of progression of liver disease in a patient comprising administering alisporivir.

11. A pharmaceutical composition comprising alisporivir for use according to any of the claims 1 to 7.
